# FASCICULE DE BREVET EUROPEEN

(11) **EP 3 021 936 B1**
(45) Date de publication et mention de la délivrance du brevet: **30.01.2019**
(21) Numéro de dépôt: 14741262.1
(22) Date de dépôt: 15.07.2014
(51) Int. Cl.: A61N 1/32, A61N 1/04, A61L 29/16, A61F 13/00

(54) **DISPOSITIF DE TRAITEMENT D'UNE ULCÉRATION CUTANÉE**
VORRICHTUNG ZUR BEHANDLUNG EINES KUTANEN GESCHWÜRS
DEVICE FOR TREATING A CUTANEOUS ULCER

(30) Priorité: 15.07.2013 FR 1356941
(43) Date de publication de la demande: 25.05.2016
(73) Titulaire: Centre Hospitalier Universitaire Grenoble, 38700 La Tronche (FR); Université Grenoble Alpes, 38400 Saint-Martin-d'Hères (FR)
(72) Inventeur: CRACOWSKI, Jean-Luc, F-38330 Saint Ismier (FR); ROUSTIT, Matthieu, F-38420 Murianette (FR); BLAISE, Sophie, F-38190 Villard Bonnot (FR)
(74) Mandataire: Hautier IP
(86) Numéro de dépôt international: PCT/EP2014/065093
(87) Numéro de publication internationale: WO 2015/007712

(56) Documents cités:
- WO-A1-2009/096822
- WO-A1-2009/124096
- US-A1- 2011 264 028
- Sophie Blaise ET AL: "Développement de la iontophorèse comme axe thérapeutique des atteintes microcirculatoires dans la sclérodermie systémique.", , 10 novembre 2011 (2011-11-10), XP055094083, Extrait de l'Internet: URL:http://tel.archives-ouvertes.fr/docs/0 0/65/00/40/PDF/20073_BLAISE_2011_archivage .pdf [extrait le 2013-12-18]

## Description

### DOMAINE DE L'INVENTION

La présente invention concerne un dispositif de traitement d'une ulcération cutanée, notamment liée à la sclérodermie systémique.

### ARRIERE PLAN DE L'INVENTION

La sclérodermie systémique est une maladie rare (touchant environ 50 à 300 patients par million d'habitants), à prédominance féminine (de l'ordre de 3 à 14 femmes pour un homme) et particulièrement invalidante.

Cette maladie est caractérisée par une fibrose cutanée et viscérale associée à une atteinte microvasculaire diffuse et la présence d'auto-anticorps dirigés contre des antigènes cellulaires.

Pour une description générale détaillée de cette maladie, on pourra se référer à la revue de Gabrielli et al [1].

La sclérodermie se décompose en deux principales catégories :
- la sclérodermie cutanée limitée, dans laquelle la fibrose se limite essentiellement aux mains, aux bras et au visage.
- la sclérodermie cutanée diffuse, maladie à progression rapide qui affecte une grande partie de la peau et des organes internes.

Il existe également des formes sans atteinte cutanée et des formes frontières avec d'autres connectivités.

La dysfonction vasculaire est un élément clé dans la pathogénie de cette maladie, précédant la fibrose.

On pourra à cet égard se référer à la revue générale détaillée de Trojanowska [2].

Cette dysfonction concerne principalement la microcirculation mais les artères de conductance sont aussi atteintes.

La microcirculation présente des anomalies de structure et de fonction, qui sont interdépendantes.

Cette micro-angiopathie est caractérisée sur un plan structural par une raréfaction capillaire, le développement de méga-capillaires et une oblitération vasculaire.

Sur un plan fonctionnel, la dysfonction microvasculaire se manifeste par un phénomène de Raynaud pouvant s'associer à des troubles trophiques cutanés ainsi qu'à une hypertension artérielle pulmonaire.

Le phénomène de Raynaud, qui peut être primaire ou secondaire, est le syndrome clinique correspondant à une vasoconstriction exagérée des microvaisseaux des doigts et/ou des orteils et/ou des oreilles en réponse à un stress environnemental (froid, plus rarement humidité) ou émotionnel.

Le phénomène de Raynaud de la sclérodermie se distingue du phénomène de Raynaud primaire par des crises plus sévères, fréquentes, touchant plus fréquemment d'autres territoires que les mains : nez, langue, oreilles, orteils.

Peu de classes thérapeutiques sont efficaces dans la prise en charge thérapeutique du phénomène de Raynaud lié à une sclérodermie.

Les ulcères digitaux sont la complication la plus sévère et la plus invalidante de la sclérodermie systémique.

Leur fréquence est estimée à 43% dans les formes cutanées limitées et à 51% dans les formes cutanées diffuses.

La physiopathologie fait intervenir une ischémie vasculaire et des facteurs mécaniques liés à une peau scléreuse sous tension.

Le traitement des ulcérations digitales de la sclérodermie systémique est d'abord préventif, dans le cadre d'une bonne hygiène cutanée et unguéale.

Sur le plan curatif, l'iloprost par voie intraveineuse est le seul médicament recommandé en cure de cinq jours dans le phénomène de Raynaud sévère.

Ce médicament a montré sa supériorité vis-à-vis des traitements conventionnels en cas d'ischémie digitale sévère ou de gangrène digitale.

Dans une étude randomisée contre placebo, l'iloprost apportait un bénéfice en termes de fréquence de crises de Raynaud et de délai de cicatrisation des ulcérations.

Utilisé dans une étude ouverte sous la forme d'une perfusion par semaine toutes les six semaines sur une période de douze mois, l'iloprost par voie intraveineuse apporte un bénéfice sur le phénomène de Raynaud et pourrait avoir un effet sur l'évolution de la maladie, comme en témoigne l'amélioration des scores cutanés ; en revanche, l'effet sur la sclérodactylie reste controversé.

Le bosentan, antagoniste non réceptif des récepteurs de l'endothéline ETA et ETB, a démontré dans le cadre de l'étude RAPIDS-2 qu'il réduisait significativement le risque de développement de nouveaux ulcères.

Cependant, le bosentan n'a montré aucun bénéfice sur la cicatrisation des ulcères préexistants et ne dispose par conséquent d'une autorisation de mise sur le marché en Europe que dans la prévention des ulcérations digitales chez des patients atteints de sclérodermie systémique ayant des ulcères digitaux actifs récidivants.

Il est à noter que le bosentan n'améliore pas le phénomène de Raynaud.

Des études sont en cours actuellement avec le sildénafil dans les ulcérations digitales.

La sympathectomie digitale est proposée par certains auteurs dans les cas les plus sévères.

En synthèse, le phénomène de Raynaud de la sclérodermie est une source de gêne fonctionnelle sévère tandis que les ulcérations digitales de la sclérodermie sont un phénomène fréquent et extrêmement invalidant, pouvant aller jusqu'à l'amputation d'un ou de plusieurs doigts et représentant la première source de handicap fonctionnel chez ces patients.

La prise en charge thérapeutique des manifestations vasculaires périphériques en 2012 est limitée et seulement partiellement efficace (seulement 3 recommandations de grade A dans les recommandations de L'EULAR (European League Against Rhumatism) : inhibiteurs calciques pour la prise en charge du phénomène de Raynaud, antagonistes de l'endothéline par voie orale (bosentan) indiqués en prévention de l'apparition d'ulcérations digitales, analogues de la prostacycline par voie intraveineuse (iloprost) en curatif au prix d'effets indésirables très fréquents (céphalées, bouffées vasomotrices, nausée, vomissements, douleurs maxillaires, myalgies).

Sur un plan pharmacologique, la difficulté de la prise en charge du phénomène de Raynaud et des ulcérations digitales de la sclérodermie est que la diffusion tissulaire des rares médicaments administrés par voie systémique ciblant la microangiopathie est limitée par la raréfaction capillaire des tissus atteints.

Cette limitation de la diffusion est difficilement compensable par une augmentation des doses administrées compte-tenu des effets indésirables systémiques fréquents de l'iloprost.

Des travaux conduits sur des rats puis sur des volontaires sains ont montré l'effet du tréprostinil (Remodulin®), qui est un analogue de la prosacycline, appliqué par iontophorèse cathodale, sur une augmentation du flux sanguin cutané.

L'iontophorèse consiste à appliquer sur la peau un courant de faible intensité (de 20 à 100 µA le plus souvent) à travers une capsule ou une éponge contenant un médicament ionisé, afin de provoquer une migration transcutanée de ce médicament.

En fonction des caractéristiques de la molécule, du tissu cible et des concentrations utilisées, l'effet pharmacodynamique pourra être limité au derme, alors que dans d'autres cas la molécule diffusera dans l'organisme avec ou sans action systémique en fonction des concentrations atteintes.

Cette modalité de délivrance de médicaments est non invasive et présente plusieurs avantages par rapport à l'administration transdermique traditionnelle passive : libération plus rapide du médicament dans la peau, meilleur contrôle de la dose administrée, capacité de diffusion dermique de molécules chargées ou de macromolécules.

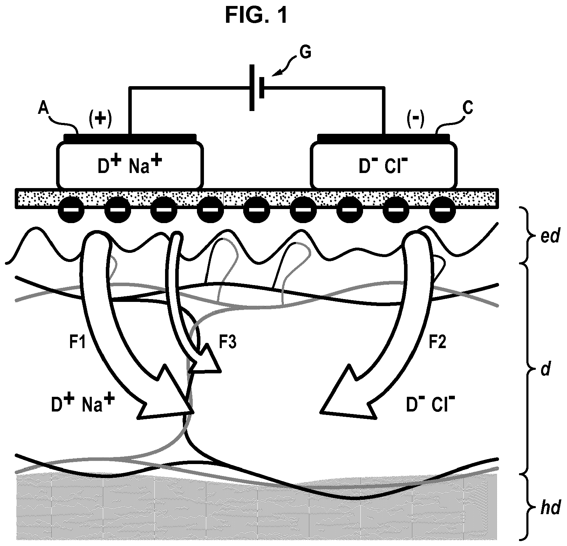

La figure 1 illustre schématiquement le principe d'un transport iontophorétique.

Le dispositif d'iontophorèse comprend deux électrodes : une anode A et une cathode C appliquées sur la peau d'un patient et connectées à un générateur de courant électrique G.

Deux mécanismes sont impliqués dans le transport iontophorétique.

D'une part, l'électromigration est le mouvement des ions à travers une membrane (ici la peau) sous l'influence directe du champ électrique.

Les médicaments chargés négativement vont être repoussés dans la peau sous la cathode, tandis que le transfert de médicaments chargés positivement se produit au niveau de l'anode.

Le second mécanisme est appelé électro-osmose, ce qui peut être schématisé comme le débit volumétrique induit par le passage du courant.

Comme le point isoélectrique (pl) de la peau humaine est d'environ 4 à 4,5, ce qui est inférieur au pH dans des conditions physiologiques, la peau sera chargée négativement.

L'application d'un champ électrique au travers de la peau aura pour effet de favoriser la circulation de cations.

Par conséquent, le débit volumique est dirigé dans le sens anode-cathode, ce qui facilite le transport de médicaments chargés positivement.

L'électro-osmose permet également la diffusion des molécules neutres par iontophorèse anodique.

La part respective du transfert par électromigration ou électro-osmose dépend fortement des propriétés physico-chimiques des molécules et de la polarité du courant appliqué.

Comme illustré sur la figure 1, les médicaments chargés positivement (D +) migrent sous l'anode tandis que les médicaments chargés négativement (D-) migrent sous la cathode.

L'épiderme est désigné par le repère ed, le derme par le repère d et l'hypoderme par le repère hd.

Les flèches F1 et F2 représentent l'électromigration anodique et cathodique, respectivement, tandis que la flèche F3 représente l'électro-osmose.

Les inventeurs ont tout d'abord testé plusieurs molécules candidates avec différentes polarités de courant (anodal et cathodal) chez l'animal, ces molécules étant contenues dans une solution liquide en contact direct avec la peau de l'animal, et ont démontré que le tréprostinil entraînait chez le rat une vasodilatation cutanée après 20 minutes d'iontophorèse cathodale à 100 µA [3].

Ces données précliniques suggèrent un effet concentration-dépendant persistant au moins 60 minutes après la fin de l'iontophorèse.

Cet effet est spécifique de l'iontophorèse cathodale, aucun effet sur le flux sanguin cutané n'étant observé sans courant et en courant anodal.

Aucun signe de toxicité locale n'a été observé, cliniquement et après biopsies cutanées systématiques.

Une étude clinique a ensuite inclus 20 volontaires sains.

Ces sujets ont reçu 3 concentrations de tréprostinil par iontophorèse cathodique continue pendant 20 minutes sur l'avant-bras et une iontophorèse de contrôle par NaCl 0.9%.

Le flux sanguin cutané a été quantifié par imagerie laser Doppler.

Le tréprostinil à 250 µM a induit une augmentation de la conductance vasculaire cutanée (AUC80 min (31 897 ± 24 390% BL.min) par rapport au contrôle NaCl 0,9% (P <0.005), tréprostinil 2,5 µM (P <0.005), et tréprostinil 25 µM (P <0.005) [4].

Cela a été confirmé lorsque le flux a été enregistré pour un maximum de 10 heures.

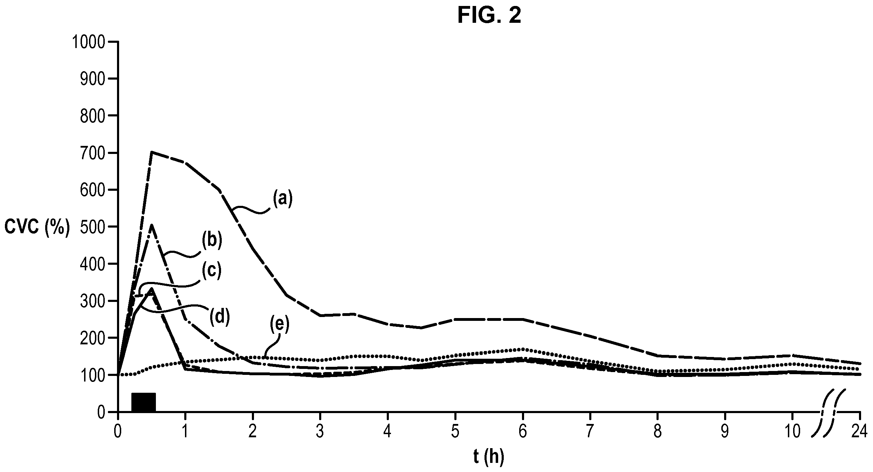

La figure 2 illustre l'effet sur l'avant-bras de l'iontophorèse (20 minutes, 20 µA, représentée par le rectangle plein) de tréprostinil 250 µM (courbe (a)), 25 µM (courbe (b)) et 2,5 µM (courbe (c)), versus NaCl 0,9% (courbe (d)) en fonction du temps t.

La courbe (e) présente l'effet du tréprostinil 250 µM appliqué sans iontophorèse.

Cet effet est exprimé en conductance vasculaire cutanée CVC (pourcentage de la ligne de base) enregistrée jusqu'à 24 heures.

Pour la mise en oeuvre de l'iontophorèse sur l'avant-bras, on a placé sur la face interne de l'avant-bras de chaque patient plusieurs chambres d'iontophorèse contenant une solution liquide de tréprostinil en contact avec une cathode, et une anode placée à distance d'environ 15 cm de la cathode.

Chaque chambre d'iontophorèse présentait une surface circulaire de 1,2 cm2 et contenait 360 µl de solution de tréprostinil.

La solution liquide de tréprostinil était directement en contact avec la peau du sujet.

Les concentrations de tréprostinil testées étaient de 2,5 µM, 25 µM et 250 µM ; une solution de NaCl était également utilisée à titre de comparaison.

Cependant, les essais réalisés jusqu'à présent étaient réalisés sur des sujets sains, par conséquent sur une peau non lésée, et en outre sur une région du corps qui n'est pas représentative de l'emplacement des ulcérations sclérodermiques.

En particulier, la face interne de l'avant-bras est large et plane, ce qui facilite l'installation de la chambre d'iontophorèse et le placement de l'anode à une distance suffisante de la cathode.

De plus, le sujet était maintenu immobile pendant les essais.

Il serait maintenant souhaitable de disposer d'un dispositif qui permette de traiter des patients sclérodermiques.

Cependant, même s'ils semblent prometteurs, les résultats des études décrites plus haut ne sont pas transposables directement au traitement des ulcérations.

Ainsi, les ulcérations ne se trouvent pas sur des zones aussi planes que la face intérieure de l'avant-bras.

En particulier, les ulcérations digitales se trouvent sur la pulpe des doigts, qui est une surface non-plane et de surface limitée.

En outre, la surface couverte par chaque chambre d'iontophorèse utilisée expérimentalement est trop faible par rapport à la surface à couvrir pour une application clinique.

D'autre part, le dispositif doit remplir également une fonction de protection des ulcérations vis-à-vis de l'environnement extérieur.

Se posent donc les problèmes suivants :
- d'une part, la tenue du dispositif d'iontophorèse sur la région à traiter pendant une longue durée, sans entraver les activités du patient,
- la couverture d'une surface correspondant à la surface de l'ulcération ou à la surface de la région dans laquelle on souhaite prévenir l'apparition d'une telle ulcération,
- l'emplacement de l'anode à une distance suffisante de la cathode pour permettre le transport iontophorétique, tout en procurant un dispositif le moins encombrant possible,
- enfin, ce dispositif étant destiné à être jeté après son utilisation, il doit être réalisable à un coût raisonnable.

Il n'existe pas à l'heure actuelle de dispositif d'iontophorèse adapté pour le traitement de lésions cutanées sur de longues durées.

Un dispositif d'iontophorèse connu, tel que le dispositif Lidosite® de la société Viterys, est destiné à l'anesthésie locale par iontophorèse de lidocaïne.

Cependant, ce dispositif doit être appliqué sur une peau non lésée ; d'autre part, à l'issue du traitement, qui est très court (de l'ordre de 10 minutes), le dispositif doit être retiré.

Un autre dispositif, nommé Ionsys™ est en cours de développement par la société Incline Therapeutics à des fins d'analgésie.

Cependant, comme le précédent, ce dispositif est destiné à être appliqué sur une peau saine.

Un but de l'invention est donc de concevoir un dispositif de traitement de lésions cutanées, notamment d'ulcérations, chez un patient atteint de sclérodermie.

WO2009096822 concerne un dispositif d'administration de médicament comprenant un élément polymère conducteur et un élément d'incorporation de substance contenant une substance.

La thèse de doctorat de Sophie Biaise soutenue le 10 novembre 2011 concerne le développement de la iontophorèse comme axe thérapeutique des atteintes microcirculatoires dans la sclérodermie systémique.

### BREVE DESCRIPTION DE L'INVENTION

Conformément à l'invention, il est proposé un dispositif de traitement d'une région du corps d'un patient présentant une ulcération cutanée, comprenant :
- un pansement primaire hydrogel, hydrocolloïde, hydrocellulaire ou hydrofibres imprégné de tréprostinil, conformable à la région à traiter,
- une électrode dite active, au contact dudit pansement primaire,
- une électrode dite passive, distante de l'électrode active,
- un générateur de courant électrique continu dont les bornes sont connectées auxdites électrodes, la cathode et l'anode formant respectivement l'électrode active et l'électrode passive d'iontophorèse, de sorte à faire migrer le tréprostinil par iontophorèse cathodale dans la région à traiter.

On rappelle qu'un pansement primaire est un pansement spécialement adapté pour être appliqué sur une plaie, la fonction d'un tel pansement étant de créer un micro-climat humide au niveau de la plaie pour favoriser sa cicatrisation tout en formant une barrière bactériologique. Un tel pansement est donc perméable aux échanges gazeux, imperméable aux liquides, capable de drainer les exsudats, et stérile, sans adhérer à la plaie. Le choix du type de pansement parmi les pansements hydrogels, hydrocolloïdes, hydrocellulaires ou hydrofibres est effectué par le praticien en fonction de la nature de la plaie à traiter.

De manière particulièrement avantageuse, la concentration du tréprostinil imprégnant le pansement primaire est comprise entre 0,25 mM et 25 mM.

Selon une forme d'exécution, l'électrode active est constituée d'une encre conductrice déposée sur le pansement primaire.

De préférence, le générateur de courant électrique continu est adapté pour générer un courant continu présentant une intensité comprise entre 20 µA/cm² et 100 µA/cm².

De manière particulièrement avantageuse, ledit générateur de courant électrique continu est porté par un support adapté pour être fixé sur la peau du patient.

Selon une forme d'exécution de l'invention, ledit générateur de courant électrique continu est solidaire du pansement primaire et des électrodes active et passive.

Selon un mode de réalisation particulier, le dispositif comprend un pansement secondaire conformable à la région à traiter, ledit pansement secondaire supportant le pansement primaire en contact avec l'électrode active, l'électrode passive et le générateur de courant électrique continu.

Selon un mode de réalisation, le dispositif comprend un module comprenant l'électrode passive et le générateur de courant électrique continu, ledit module étant distinct du pansement primaire et adapté pour être connecté à l'électrode active.

De manière avantageuse, la surface du pansement primaire est comprise entre 5 cm² et 100 cm².

Selon une forme d'exécution préférée de l'invention, le dispositif se présente sous une forme adaptée pour être enfilée sur un doigt du patient, le pansement primaire et l'électrode active étant adaptés pour se conformer à la pulpe dudit doigt et l'électrode passive étant adaptée pour se conformer à la face externe du doigt opposée à la pulpe.

Le dispositif peut en outre comprendre un pansement secondaire conformable à la région à traiter, destiné à recouvrir le pansement primaire.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma de principe d'un transport iontophorétique,
- la figure 2 présente l'effet sur la conductance vasculaire cutanée de l'avant-bras d'un sujet sain de différentes concentrations de tréprostinil sous forme d'une solution liquide appliqué par iontophorèse,
- la figure 3 est une image obtenue par Laser Speckle Contrast Imaging du flux sanguin dans la pulpe d'un index traitée par iontophorèse de tréprostinil imprégnant un pansement primaire et dans la pulpe du majeur de la même main traitée par iontophorèse de NaCl, chez un sujet sain,
- la figure 4 est une image obtenue par Laser Speckle Contrast Imaging du flux sanguin dans la pulpe d'un annulaire traitée par iontophorèse de tréprostinil imprégnant un pansement primaire et dans la pulpe du majeur de la même main traitée par iontophorèse de NaCl, chez un sujet atteint de sclérodermie,
- la figure 5 est un schéma d'un dispositif selon un mode de réalisation de l'invention destiné à être appliqué sur la pulpe d'un doigt, dans lequel les électrodes et le générateur sont intégrés au pansement primaire,
- la figure 6 est un schéma d'un dispositif selon un mode de réalisation de l'invention destiné à être appliqué sur la pulpe d'un doigt, dans lequel l'électrode passive et le générateur sont dissociés du pansement primaire,
- la figure 7 est un schéma d'un dispositif selon un mode de réalisation de l'invention destiné à être appliquée sur un membre inférieur du patient.

### DESCRIPTION DETAILLEE DE MODES DE REALISATION DE L'INVENTION

Les inventeurs ont démontré l'efficacité en termes de vasodilatation d'un traitement par iontophorèse au moyen d'un pansement primaire imprégné de tréprostinil.

Ils ont également montré que le tréprostinil était présent (quantification en chromatographie liquide haute performance couplée à la spectrométrie de masse en tandem dans du liquide de dialysat dermique recueilli par les fibres de microdialyse) dans le derme de l'avant-bras pendant une durée moyenne allant jusqu'à 8 heures.

La mise en oeuvre du tréprostinil en tant qu'imprégnation d'un pansement primaire présente l'avantage de permettre de couvrir des surfaces cutanées plus importantes.

Il n'était cependant pas évident que les résultats obtenus par iontophorèse de tréprostinil sous la forme d'une solution liquide en contact direct avec la peau du patient puissent être obtenus avec la forme de mise en oeuvre conforme à l'invention.

En premier lieu, le fait que l'iontophorèse entraine une vasodilatation avec une solution liquide n'implique pas le même effet lorsque le médicament est imprégné sur un pansement primaire

Par ailleurs, on pouvait craindre que l'imprégnation du pansement n'empêche - ou tout au moins entrave fortement - la migration des molécules vers la région à traiter et ce, d'autant plus que la peau des sujets atteints de sclérodermie est sensiblement plus épaisse que celle des sujets sains.

D'autre part, l'efficacité du tréprostinil sous forme de solution liquide a été démontrée dans des conditions particulières de concentration et de courant appliqué, qui ne sont pas transposables à un pansement imprégné.

En effet, le champ électrique appliqué étant différent, la diffusion des molécules engendrée par ce champ électrique est nécessairement différente.

Une efficacité inférieure à celle de l'iontophorèse du tréprostinil sous forme d'une solution liquide était donc attendue.

Par ailleurs, l'application d'un courant électrique est susceptible de causer un inconfort voire des lésions au patient, si la densité de courant (intensité par unité de surface) dudit courant est trop élevée.

Parmi les risques associés à l'iontophorèse, on peut citer des sensations de fourmillement, de prurit, de chaleur, un érythème, voire une brûlure.

Cependant, les inventeurs sont parvenus à définir des conditions de mise en oeuvre de l'iontophorèse qui permettent, même avec un pansement primaire imprégné de tréprostinil, d'obtenir chez le patient les effets observés dans les expérimentations préalables, sans engendrer d'effets indésirables notables chez les patients.

Un autre obstacle qui a été franchi par les inventeurs est l'obtention d'un effet pharmacologique du pansement sur une peau scléreuse, alors que les dispositifs d'iontophorèse ont jusqu'à ce jour toujours été appliqués sur une peau saine. De ce fait, les dispositifs d'iontophorèse existants mettent en oeuvre des supports, par exemple sous forme d'éponges, qui ne sont pas adaptés - en termes de sécurité - pour être mis en contact avec des plaies.

Cette application a nécessité la mise au point d'un pansement primaire spécifiquement adapté au contact d'une peau lésée.

Enfin, le dispositif conforme à l'invention est destiné à être porté par le patient pendant une longue durée (typiquement, au moins quelques heures).

Or, le fait que la peau du patient soit en contact prolongé avec une substance active est susceptible d'impliquer un passage systémique du tréprostinil.

Les inventeurs ont donc dosé le tréprostinil au niveau plasmatique en chromatographie liquide haute performance couplée à la spectrométrie de masse en tandem après une iontophorèse de 20 min au niveau de l'avant-bras, et de 2 heures au niveau des doigts.

La quantification systémique permet de démontrer un passage systémique négligeable.

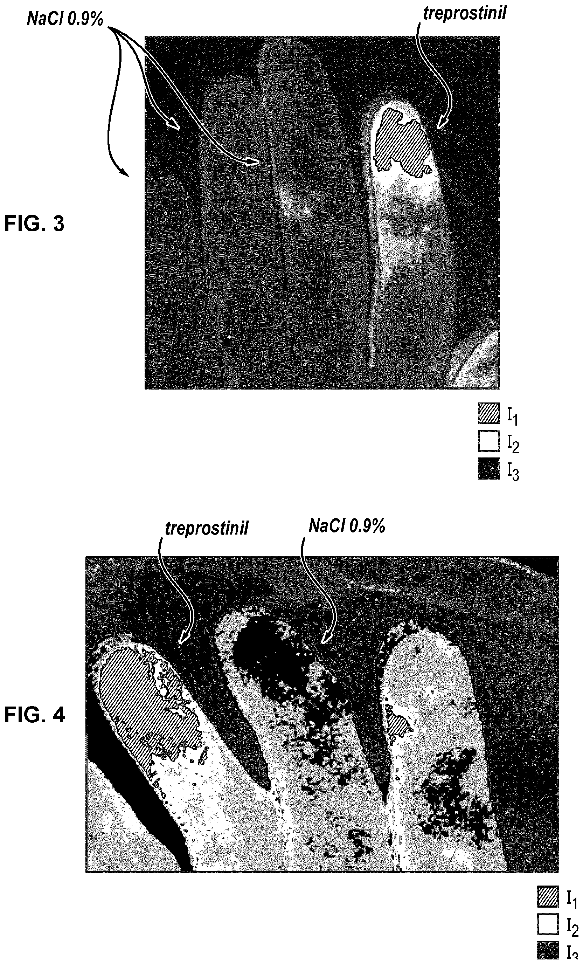

Les figures 3 et 4 mettent en évidence l'effet vasomoteur du tréprostinil imprégnant un pansement primaire appliqué sur la pulpe d'un doigt, respectivement chez un sujet sain et chez un sujet atteint de sclérodermie.

La figure 3 reproduit une image obtenue par Laser Speckle Contrast Imaging du flux sanguin dans la pulpe d'un index traitée par iontophorèse de tréprostinil imprégnant un pansement primaire et dans la pulpe du majeur de la même main traitée par iontophorèse de NaCl 0,9% imprégnant un autre pansement primaire, chez un sujet sain.

L'iontophorèse a été réalisée dans les conditions suivantes : 240 mC/cm², courant continu.

Deux électrodes Ag-AgCl de 7,2 cm ² (logel, lomed Inc., Salt Lake City, UT, USA) ont été imbibées de 1,5 ml de tréprostinil ou d'une solution de NaCl (en double aveugle).

Ces électrodes contenant un médicament ont été connectées à la cathode, tandis que l'anode était reliée à des électrodes passives fixées à une distance de 10 cm.

Des générateurs basse tension pilotés par ordinateur ont été utilisés (PF 751 Perilont USB Power Supply, Perimed, Järfälla, Suède).

La tension a été enregistrée en continu pour estimer la résistance de la peau.

Sur le majeur, on n'observe aucun effet de l'iontophorèse.

Sur l'index, les différentes régions, qui sont représentées par une échelle de couleurs sur les images réelles montrent les différents débits de flux sanguins au niveau de la pulpe traitée par le tréprostinil.

La région hachurée I₁ correspond à un flux sanguin élevé, la région blanche I₂ correspond à un flux sanguin modéré et la région foncée I₃ correspond à un flux sanguin faible.

La figure 4 reproduit une image obtenue par Laser Speckle Contrast Imaging du flux sanguin dans la pulpe d'un annulaire traitée par iontophorèse de tréprostinil imprégnant un pansement primaire et dans la pulpe du majeur de la même main traitée par iontophorèse de NaCl 0,9%, chez un sujet atteint de sclérodermie.

La variation du flux sanguin est représentée de la même manière que sur la figure 3.

Il s'agit donc de la première démonstration de passage transcutané de tréprostinil au travers d'une peau lésée, c'est-à-dire scléreuse.

Comme sur la figure 3, on observe un effet vasomoteur marqué sur la pulpe du doigt traité par iontophorèse du tréprostinil (annulaire), et aucun effet sur la pulpe du doigt traité avec la solution de NaCl de référence (majeur).

Une étude a été conduite sur 12 patients atteints de sclérodermie et a permis de démontrer que l'iontophorèse cathodale de tréprostinil avec une concentration de 250 µM, en courant continu, à 240 mC/cm², est bien tolérée et qu'elle est associée à une augmentation du flux sanguin cutané.

Les échantillons de plasma prélevés chez chaque patient 30 minutes après la fin de l'iontophorèse présentaient une concentration en tréprostinil inférieure à 1,8 pg / mL.

D'une manière générale, le dispositif de traitement comprend un pansement primaire imprégné de tréprostinil.

Le pansement primaire est destiné à être appliqué directement sur la peau lésée (ulcérée) du patient, et est donc en un matériau adapté à cette application.

De manière avantageuse, le pansement primaire est choisi parmi l'un des quatre types de pansements suivants :
- un pansement hydrogel,
- un pansement hydrocolloïde,
- un pansement hydrocellulaire,
- un pansement hydrofibres.

Ces différents types de pansement existent actuellement sur le marché et l'homme du métier est à même de sélectionner, parmi les pansements disponibles, celui qui est le plus adapté à l'application visée.

On ne décrira donc pas en détail ici la composition de ces différents pansements, celle-ci étant bien documentée dans la littérature scientifique.

Un pansement hydrogel est bien adapté à une plaie sèche, fibrineuse ou nécrotique, tandis qu'un pansement hydrocolloïde, hydrocellulaire ou hydrofibres est préféré en phase de bourgeonnement.

Dans tous les cas, le pansement primaire présente une flexibilité suffisante pour lui permettre de se conformer à la région à traiter, c'est-à-dire d'épouser la forme de ladite région pour être en contact avec sensiblement toute la surface de la région à traiter.

D'autre part, la flexibilité du pansement primaire permet de ne pas exercer de pression excessive sur la peau, qui induirait un risque de lésion cutanée.

Selon les cas, le pansement primaire peut se présenter sous forme plane et être conformé par l'utilisateur à la région à traiter lors de sa mise en place sur ladite région.

De manière alternative, le dispositif de traitement peut être fourni à l'utilisateur avec le pansement primaire préalablement conformé à la région à traiter.

Par exemple, si le dispositif est destiné à traiter une pulpe digitale, le dispositif se présente sous la forme d'un doigtier dont le pansement occupe au moins la portion destinée à être en contact avec la pulpe.

La surface du pansement primaire dépend de la région à traiter.

On choisit la surface du pansement primaire en fonction de la surface des plaies à traiter, tout en limitant le risque d'exposition systémique au tréprostinil.

Typiquement, le pansement primaire présente une surface comprise entre 5 cm² et 100 cm².

Quel que soit le type de pansement primaire choisi, celui-ci est imprégné d'une solution liquide de tréprostinil.

Le tréprostinil est un analogue synthétique de la prostacycline, actuellement commercialisé sous le nom Remodulin®.

Le volume de tréprostinil dépend de la nature, de la surface et de l'épaisseur du pansement primaire.

L'objectif est que le volume de solution de tréprostinil sature le pansement afin de permettre un contact cutané de la molécule.

A cet effet, on peut envisager soit l'imprégnation en temps réel (la solution étant déposée sur le pansement juste avant l'application), soit l'imprégnation préalable intégrée (pansement « serti » de tréprostinil), dans ce dernier cas sous réserve de vérification de la stabilité du produit.

La concentration de tréprostinil est choisie parmi les concentrations dont les inventeurs ont mis en évidence l'efficacité sous forme imprégnée.

Ainsi, la concentration du tréprostinil est de préférence comprise entre 0,25 mM et 25 mM.

Le dispositif de traitement comprend en outre une électrode active au contact du pansement primaire, du côté opposé à celui destiné à être au contact de la peau du patient.

Cette électrode active est conçue pour pouvoir être connectée à une borne d'un générateur de courant électrique.

Comme indiqué plus bas, le générateur de courant électrique peut faire partie intégrante du dispositif de traitement, mais il peut également être distinct de celui-ci.

En tout état de cause, l'électrode active comprend un connecteur permettant son raccordement à une borne d'un tel générateur de courant électrique.

S'agissant d'iontophorèse cathodale, l'électrode active est destinée à constituer la cathode du dispositif.

De préférence, l'électrode est réalisée en un matériau qui permette de minimiser l'encombrement du dispositif et également de ne pas pénaliser la conformabilité du pansement à la région à traiter.

L'épaisseur de l'électrode active est avantageusement inférieure ou égale à 500 µm.

Selon un mode de réalisation avantageux, l'électrode active est constituée d'une encre conductrice (par exemple en argent) déposée sur le pansement primaire.

La surface de l'électrode active doit être suffisamment large pour, d'une part, couvrir la surface du pansement imbibé de tréprostinil et, d'autre part, permettre une basse densité de charge électrique n'entraînant pas de réaction locale.

Le dispositif de traitement comprend en outre une électrode passive, qui est agencée à distance de l'électrode active.

De préférence, on fait en sorte que la distance entre l'électrode active et l'électrode passive soit la plus grande possible.

L'électrode passive est destinée à constituer l'anode du dispositif d'iontophorèse.

Elle est donc conçue pour être connectée à l'autre borne du générateur de courant continu mentionné plus haut.

Selon un mode de réalisation, l'électrode passive fait partie intégrante du dispositif, c'est-à-dire qu'elle est fournie à l'utilisateur solidaire du pansement primaire et de l'électrode active, ce qui facilite la mise en place du dispositif sur le patient.

De manière alternative, l'électrode passive est fournie séparée du pansement primaire et de l'électrode active.

Dans ce cas, l'utilisateur met en place successivement le pansement primaire et l'électrode passive, puis réalise la connexion avec le générateur de courant continu.

Pour minimiser l'encombrement du dispositif de traitement, l'électrode passive est de préférence en un matériau suffisamment fin et flexible pour pouvoir épouser la forme de la région sur laquelle elle est appliquée.

L'électrode passive est par exemple formée sur un support adhésif permettant de la fixer sur la peau du patient.

L'électrode passive est constituée d'un métal souple et d'un adhésif, et doit être conformable à l'utilisation.

La surface de l'électrode passive est si possible équivalente à celle de l'électrode active de façon que la densité de charge reste modeste et n'entraîne pas de picotements lors de la mise en route du générateur.

Pour diffuser le tréprostinil dans la région à traiter, on applique, au moyen d'un générateur approprié, un courant continu présentant une intensité typiquement comprise entre 20 µA/cm² et 100 µA/cm².

Selon un mode de réalisation, le générateur de courant électrique continu est distinct du dispositif de traitement : il peut s'agir d'un générateur présent dans le commerce, auquel un utilisateur connecte chacune des électrodes active et passive.

Dans ce cas, la taille du générateur importe peu.

Compte tenu de la contrainte que représente le fait pour l'utilisateur d'être relié à un générateur fixe, le traitement par iontophorèse peut être mis en oeuvre de manière discontinue, c'est-à-dire que l'on n'applique un courant électrique que pendant des plages temporelles limitées puis l'on déconnecte le générateur pour permettre au patient de vaquer à ses occupations.

Cette modalité est permise par le fait que l'effet du tréprostinil se poursuit pendant une certaine durée après la fin de la mise en oeuvre de l'iontophorèse, comme le montre la figure 2 et comme le montre également la persistance dans le derme de tréprostinil jusqu'à 8 heures après l'iontophorèse.

Pour maximiser l'efficacité du traitement, on fait en sorte que les plages temporelles d'application du courant électrique continu soient suffisamment proches les unes des autres pour éviter une diminution trop importante de l'effet du tréprostinil.

Selon un autre mode de réalisation, le générateur de courant électrique continu fait partie du dispositif de traitement, c'est-à-dire qu'il est adapté pour être porté par le patient.

Ceci permet d'une part de permettre au patient de vaquer librement à ses activités, et d'autre part d'appliquer le traitement sans interruption, afin de bénéficier de l'effet maximal du tréprostinil.

Dans ce cas, compte tenu des contraintes d'encombrement et de confort d'utilisation du dispositif, on emploie un générateur de courant électrique miniaturisé.

Par sécurité, le générateur comprend un système d'arrêt automatique lorsqu'une tension excessive est observée.

Le générateur peut de préférence être alimenté par une pile ou une batterie.

Avantageusement, le générateur peut en outre comprendre un témoin de charge et un témoin de qualité du circuit électrique.

Ces fonctionnalités sont connues en elles-mêmes et ne seront donc pas décrites en détail dans le présent texte.

Le générateur de courant électrique peut être fourni sur un même support que le pansement primaire et les électrodes active et passive, ce qui permet de réaliser les connexions lors de la fabrication du dispositif et de fournir à l'utilisateur un dispositif prêt à l'emploi, ne nécessitant aucun assemblage ni connexion.

La facilité d'utilisation du dispositif est alors maximale.

Selon une autre forme de mise en oeuvre, le générateur de courant électrique continu miniature est porté par un support adapté pour être fixé sur la peau du patient, et qui supporte également, de préférence, l'électrode passive.

Ainsi, l'utilisateur reçoit le dispositif de traitement en deux parties (la première comprenant le pansement primaire et l'électrode active, la seconde étant un module comprenant le générateur et l'électrode passive), et il suffit de connecter le générateur à l'électrode active pour en permettre le fonctionnement.

Ceci permet, le cas échéant, de placer l'électrode active et l'électrode passive à une grande distance l'une de l'autre, sans imposer qu'elles soient portées par un même support qui serait alors encombrant.

Toutes les parties du dispositif destinées à être en contact avec la peau du patient, et en particulier le pansement primaire, sont stériles.

Après la fabrication du dispositif, celui-ci est donc conditionné dans un emballage permettant de maintenir cette stérilité.

Par ailleurs, le dispositif est à usage unique, c'est-à-dire qu'il est jeté après la mise en oeuvre d'un traitement par iontophorèse et/ou lorsqu'il ne reste plus suffisamment de tréprostinil dans le pansement primaire.

Enfin, compte tenu de la possibilité d'un stockage du dispositif préalablement à son utilisation, les composants et notamment le tréprostinil, sont choisis pour avoir une durée de péremption suffisamment longue, par exemple d'au moins un an.

Selon une forme d'exécution de l'invention, le pansement primaire est lui-même conçu non seulement pour servir de support au tréprostinil et à l'électrode active, mais aussi pour assurer la protection de la région traitée vis-à-vis de l'environnement extérieur.

Le pansement primaire remplit alors également une fonction de pansement secondaire, qui peut être assurée par exemple par une couche extérieure imperméable aux bactéries, limitant les souillures et éventuellement amortissant les chocs.

Tel peut être le cas d'un pansement hydrocolloïde, hydrocellulaire ou hydrofibres.

De manière alternative, le dispositif de traitement peut comprendre un pansement secondaire distinct du pansement primaire, destiné à recouvrir ledit pansement primaire une fois celui-ci mis en place sur la région à traiter. Tel est le cas notamment lorsque le pansement primaire est un hydrogel.

Ce pansement secondaire doit permettre de limiter les contaminations et d'amortir les chocs.

Dans les cas où la région à traiter présente une courbure prononcée, le pansement secondaire peut en outre aider à maintenir le pansement primaire au contact de la peau pour permettre une diffusion optimale du tréprostinil.

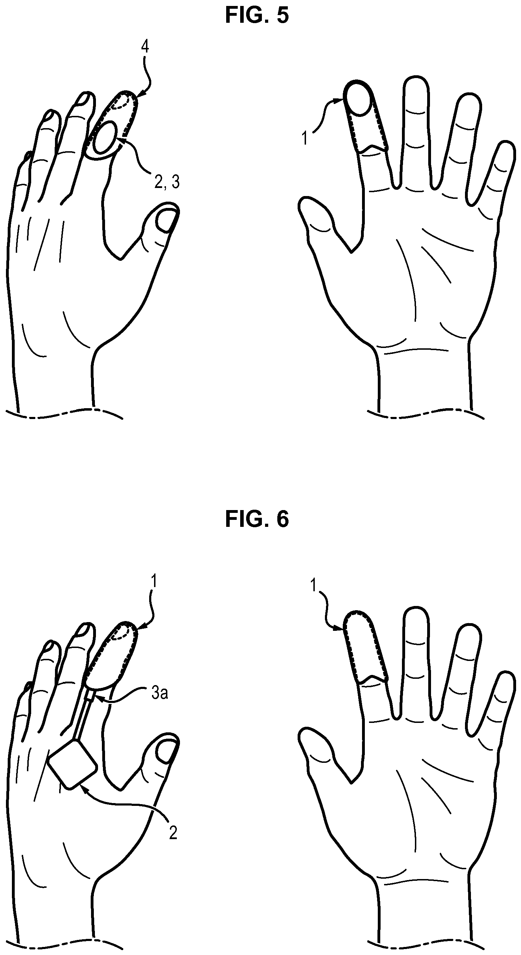

La figure 5 illustre un mode de réalisation de l'invention spécifiquement adapté au traitement de la pulpe digitale d'un patient, dans lequel les électrodes active et passive et le générateur sont solidaires du pansement primaire.

Le pansement primaire, qui est recouvert de l'électrode active 1, est agencé au contact de la pulpe du doigt à traiter.

L'électrode passive 2 est agencée au contact de la face externe du doigt, sans être en contact avec le pansement primaire.

Le générateur 3 est agencé dans la même région que l'électrode passive 2.

Le pansement primaire, les électrodes et le générateur sont rendus solidaires les uns des autres par un pansement secondaire 4 présentant la forme d'un doigtier, auquel ils sont fixés.

Pour mettre en oeuvre le traitement, l'utilisateur enfile simplement le dispositif à l'extrémité du doigt et met en marche le générateur de courant électrique continu pour faire diffuser le tréprostinil.

Le courant électrique continu peut être appliqué pendant toute la durée du port du dispositif.

Une fois que le traitement est terminé (c'est-à-dire typiquement au bout d'un délai compris entre 20 minutes et 2 heures) si le dispositif n'est pas utilisé comme un pansement, ou lors du changement de pansement si le dispositif fait office de pansement primaire et/ou secondaire (par exemple tous les 2 jours), il suffit de retirer le dispositif du doigt et de le jeter.

La figure 6 est un schéma d'un dispositif selon un mode de réalisation de l'invention destiné à être appliqué sur la pulpe d'un doigt, dans lequel l'électrode passive et le générateur sont dissociés du pansement primaire.

Dans ce cas, le pansement primaire et l'électrode active 1 se présentent sous la forme d'un doigtier destiné à être enfilé à l'extrémité du doigt, et pouvant couvrir toute la circonférence du doigt.

L'électrode passive 2 et le générateur de courant électrique continu (non schématisé ici) sont quant à eux fixés sur une autre région de la main - par exemple, sur cette figure, sur le dos de la main, à distance du doigtier.

Un connecteur 3a permet de connecter l'électrode active 1 au générateur de courant électrique continu.

Comme dans le précédent mode de réalisation, le courant électrique continu peut être appliqué pendant toute la durée du port du dispositif.

Une fois que le traitement est terminé, il suffit de retirer le pansement primaire et l'électrode active du doigt et de les jeter.

Le cas échéant, l'électrode passive et le générateur de courant électrique continu peuvent être conservés pour un traitement ultérieur.

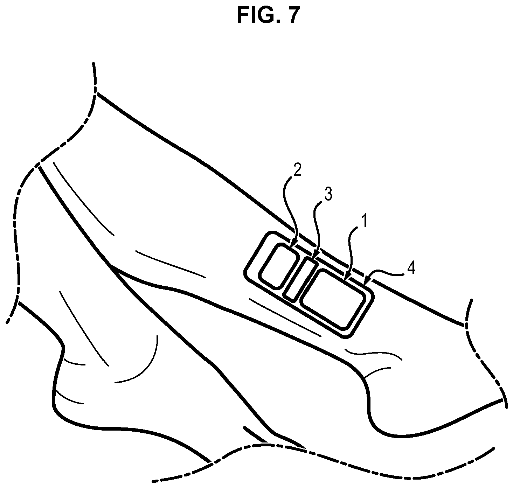

La figure 7 est un schéma d'un dispositif selon un mode de réalisation de l'invention destiné à être appliquée sur un membre inférieur du patient.

Ce dispositif peut en particulier être utilisé pour traiter et/ou prévenir les plaies du diabète.

Comme on peut le voir sur cette figure, la région traitée est sensiblement plane, ce qui autorise l'utilisation d'un pansement primaire sensiblement plan.

La surface dudit pansement peut également être plus élevée que dans le cas du traitement de la pulpe digitale.

Dans cette forme d'exécution, le dispositif de traitement intègre à la fois le pansement primaire et l'électrode active 1, l'électrode passive 2 et le générateur de courant électrique continu 3 qui est agencé entre les électrodes 1 et 2.

La solidarisation de ces différents éléments est assurée par un support adhésif 4 qui remplit en outre la fonction de pansement secondaire.

En ce qui concerne les indications thérapeutiques du dispositif décrit plus haut, la sclérodermie est particulièrement ciblée.

Cependant, cette application n'est pas limitative et le dispositif pourra également être utilisé pour traiter et/ou prévenir d'autres types de lésions cutanées, telles que les ulcérations de jambe d'origine macro ou microvasculaire, comme les plaies du diabète par exemple.

Le dispositif peut par ailleurs être appliqué sur toute région du corps du patient selon la pathologie à traiter ; si les doigts (dans le cas de la sclérodermie) ou les membres inférieurs (dans le cas des plaies du diabète) sont particulièrement concernés, l'application du dispositif n'est pas limitée à ces régions.

### REFERENCES

[1] Gabrielli A, Avvedimento EV, Krieg T. Scleroderma. The New England journal of medicine. 2009;360:1989-2003
[2] Trojanowska M. Cellular and molecular aspects of vascular dysfunction in systemic sclerosis. Nat Rev Rheumatol. 2010;6:453-460
[3] Biaise S, Roustit M, Millet C, Ribuot C, Boutonnat J, Cracowski JL. Cathodal iontophoresis of treprostinil and iloprost induces a sustained increase in cutaneous flux in rats. British journal of pharmacology. 2011;162:557-565
[4] Biaise S, Roustit M, Hellmann M, Millet C, Cracowski JL. Cathodal iontophoresis of treprostinil induces a sustained increase in cutaneous blood flux in healthy volunteers. J Clin Pharmacol. 2012

## Revendications

1. Dispositif de traitement d'une région du corps d'un patient présentant une ulcération cutanée, comprenant :
- une électrode (1) dite active,
- une électrode (2) dite passive, distante de l'électrode active,
- un générateur de courant électrique continu dont les bornes sont connectées auxdites électrodes (1, 2), la cathode et l'anode formant respectivement l'électrode active et l'électrode passive d'iontophorèse,
ledit dispositif étant **caractérisé en ce qu'**il comprend un pansement primaire hydrogel, hydrocolloïde, hydrocellulaire ou hydrofibres imprégné de tréprostinil, conformable à la région à traiter, adapté pour être appliqué directement sur la peau du patient dans la région présentant l'ulcération, l'électrode active étant au contact dudit pansement primaire de sorte à provoquer une migration du tréprostinil par iontophorèse cathodale dans la région à traiter sous l'effet de l'application d'un courant électrique continu par le générateur.

2. Dispositif selon la revendication 1, **caractérisé en ce que** la concentration du tréprostinil imprégnant le pansement primaire est comprise entre 0,25 mM et 25 mM.

3. Dispositif selon l'une des revendications 1 ou 2, **caractérisé en ce que** l'électrode active (1) est constituée d'une encre conductrice déposée sur le pansement primaire.

4. Dispositif selon l'une des revendications 1 à 3, **caractérisé en ce que** le générateur (3) de courant électrique continu est adapté pour générer un courant continu présentant une intensité comprise entre 20 µA/cm² et 100 µA/cm².

5. Dispositif selon l'une des revendications 1 à 4, **caractérisé en ce que** ledit générateur de courant électrique continu est porté par un support adapté pour être fixé sur la peau du patient.

6. Dispositif selon l'une des revendications 1 à 5, **caractérisé en ce que** ledit générateur de courant électrique continu est solidaire du pansement primaire et des électrodes active et passive.

7. Dispositif selon l'une des revendications 1 à 6, **caractérisé en ce qu'**il comprend un pansement secondaire (4) conformable à la région à traiter, ledit pansement secondaire supportant le pansement primaire en contact avec l'électrode active, l'électrode passive et le générateur de courant électrique continu.

8. Dispositif selon l'une des revendications 1 à 6, **caractérisé en ce qu'**il comprend un module comprenant l'électrode passive (2) et le générateur de courant électrique continu (3), ledit module étant distinct du pansement primaire et adapté pour être connecté à l'électrode active.

9. Dispositif selon l'une des revendications 1 à 8, **caractérisé en ce que** la surface du pansement primaire est comprise entre 5 cm² et 100 cm².

10. Dispositif selon l'une des revendications 1 à 9, **caractérisé en ce qu'**il se présente sous une forme adaptée pour être enfilée sur un doigt du patient, le pansement primaire et l'électrode active étant adaptés pour se conformer à la pulpe dudit doigt et **en ce que** l'électrode passive est adaptée pour se conformer à la face externe du doigt opposée à la pulpe.

11. Dispositif selon l'une des revendications 1 à 10, **caractérisé en ce qu'**il comprend un pansement secondaire conformable à la région à traiter, destiné à recouvrir le pansement primaire.

## Patentansprüche

1. Vorrichtung zur Behandlung eines Bereichs des Körpers eines Patienten, der ein kutanes Geschwür aufweist, umfassend:
- eine als aktiv bezeichnete Elektrode (1),
- eine als passiv bezeichnete Elektrode (2), die von der aktiven Elektrode entfernt ist,
- einen Generator von elektrischem Gleichstrom, dessen Klemmen mit den Elektroden (1, 2) verbunden sind, wobei die Kathode und die Anode jeweils die aktive Elektrode und die passive Elektrode der lontophorese bilden,
wobei die Vorrichtung **dadurch gekennzeichnet ist, dass** sie einen primären Hydrogel-, Hydrocolloid-, hydrozellulären oder Hydrofaserverband, der mit Hydroreprostinil imprägniert ist, umfasst, der an den zu behandelnden Bereich angepasst werden kann, der ausgelegt ist, um direkt auf die Haut des Patienten in dem Bereich angebracht zu werden, der das Geschwür aufweist, wobei die aktive Elektrode in Kontakt mit dem primären Verband ist, um eine Migration des Treprostinils durch kathodale lontophorese im zu behandelnden Bereich unter der Wirkung der Anwendung eines elektrischen Gleichstroms durch den Generator zu verursachen.

2. Vorrichtung nach Anspruch 1, **dadurch gekennzeichnet, dass** die Konzentration des Treprostinils, das den primären Verband imprägniert, zwischen 0,25 nM und 25 mM liegt.

3. Vorrichtung nach einem der Ansprüche 1 oder 2, **dadurch gekennzeichnet, dass** die aktive Elektrode (1) aus einer leitenden Tinte besteht, die auf dem primären Verband abgelegt ist.

4. Vorrichtung nach einem der Ansprüche 1 bis 3, **dadurch gekennzeichnet, dass** der Generator (3) von elektrischem Gleichstrom ausgelegt ist, um einen Gleichstrom zu erzeugen, der eine Intensität zwischen 20 µA/cm² und 100 µA/cm² aufweist.

5. Vorrichtung nach einem der Ansprüche 1 bis 4, **dadurch gekennzeichnet, dass** der Generator von elektrischem Gleichstrom von einer Stütze getragen wird, die ausgelegt ist, um auf der Haut des Patienten fixiert zu sein.

6. Vorrichtung nach einem der Ansprüche 1 bis 5, **dadurch gekennzeichnet, dass** der Generator von elektrischem Gleichstrom fest mit dem primären Verband und der aktiven und passiven Elektrode verbunden ist.

7. Vorrichtung nach einem der Ansprüche 1 bis 6, **dadurch gekennzeichnet, dass** sie einen sekundären Verband (4) umfasst, der an den zu behandelnden Bereich angepasst werden kann, wobei der sekundäre Verband den primären Verband in Kontakt mit der aktiven Elektrode, der passiven Elektrode und dem Generator von elektrischem Gleichtrom stützt.

8. Vorrichtung nach einem der Ansprüche 1 bis 6, **dadurch gekennzeichnet, dass** sie ein Modul umfasst, umfassend die passive Elektrode (2) und den Generator von elektrischem Gleichstrom (3), wobei das Modul verschieden vom primären Verband und ausgelegt ist, um mit der aktiven Elektrode verbunden zu sein.

9. Vorrichtung nach einem der Ansprüche 1 bis 8, **dadurch gekennzeichnet, dass** die Oberfläche des primären Verbands zwischen 5 cm² und 100 cm² liegt.

10. Vorrichtung nach einem der Ansprüche 1 bis 9, **dadurch gekennzeichnet, dass** sie eine Form aufweist, die ausgelegt ist, um über einen Finger des Patienten gestreift zu sein, wobei der primäre Verband und die aktive Elektrode ausgelegt sind, um sich an die Kuppe des Fingers anzupassen, und dadurch, dass die passive Elektrode ausgelegt ist, um sich an die äußere Seite des Fingers gegenüber der Kuppe anzupassen.

11. Vorrichtung nach einem der Ansprüche 1 bis 10, **dadurch gekennzeichnet, dass** sie einen sekundären Verband umfasst, der an den zu behandelnden Bereich angepasst werden kann, der dazu bestimmt ist, den primären Verband abzudecken.

## Claims

1. Device for treating a region of the body of a patient presenting a skin ulceration, comprising:
- an electrode (1) termed active,
- an electrode (2) termed passive, remote from the active electrode,
- an electrical direct current generator, of which the terminals are connected to said electrodes (1, 2), the cathode and the anode respectively forming the iontophoresis active electrode and the iontophoresis passive electrode,
said device being **characterised in that** it comprises a primary hydrogel, hydrocolloid, hydrocellular or hydrofibre dressing, impregnated with treprostinil, which can be shaped to the region to be treated, suitable for being applied directly on the skin of the patient in the region presenting the ulceration, the active electrode being in contact with said primary dressing so as to cause a migration of the treprostinil by cathodal iontophoresis in the region to be treated under the effect of the application of an electrical direct current by the generator.

2. Device according to claim 1, **characterised in that** the concentration of the treprostinil impregnating the primary dressing is between 0.25mM and 25mM.

3. Device according to one of claims 1 or 2, **characterised in that** the active electrode (1) is constituted of a conductive ink deposited on the primary dressing.

4. Device according to one of claims 1 to 3, **characterised in that** the electrical direct current generator (3) is suitable for generating a direct current having an intensity of between 20µA/cm² and 100µA/cm².

5. Device according to one of claims 1 to 4, **characterised in that** said electrical direct current generator is carried by a support suitable for being fixed on the skin of the patient.

6. Device according to claims 1 to 5, **characterised in that** said electrical direct current generator is secured to the primary dressing and the active and passive electrodes.

7. Device according to one of claims 1 to 6, **characterised in that** it comprises a secondary dressing (4) which can be shaped to the region to be treated, said secondary dressing supporting the primary dressing in contact with the active electrode, the passive electrode and the electrical direct current generator.

8. Device according to one of claims 1 to 6, **characterised in that** it comprises a module comprising the passive electrode (2) and the electrical direct current generator (3), said module being remote from the primary dressing and suitable for being connected to the active electrode.

9. Device according to one of claims 1 to 8, **characterised in that** the surface of the primary dressing is between 5cm² and 100cm².

10. Device according to one of claims 1 to 9, **characterised in that** it is presented in a form suitable for being threaded on a finger of the patient, the primary dressing and the active electrode being suitable for being shaped to the flesh of said finger and **in that** the passive electrode is suitable for being shaped to the outer face of the finger opposite the flesh.

11. Device according to one of claims 1 to 10, **characterised in that** it comprises a secondary dressing, which can be shaped to the region to be treated, configured to cover the primary dressing.
